(19)

Europäisches
Patentamt
European
Patent Office
Office européen
des brevets

(11) **EP 4 438 984 A1**

(12) **EUROPEAN PATENT APPLICATION**

(43) Date of publication:
**02.10.2024 Bulletin 2024/40**

(21) Application number: **23198644.9**

(22) Date of filing: **20.09.2023**

(51) International Patent Classification (IPC):
**F26B 25/00** (2006.01)

(52) Cooperative Patent Classification (CPC):
**F26B 9/10; F26B 23/007; F26B 23/028;
F26B 25/009; G01N 33/0091**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.03.2023 NO 20230344**

(71) Applicant: **Resitec AS
4617 Kristiansand (NO)**

(72) Inventors:
• **SØILAND, Anne-Karin
4623 Kristiansand (NO)**
• **MØRK, Knut
4623 Kristiansand (NO)**
• **HEINTZ, Mads Jonas Christensen
4623 Kristiansand (NO)**

(74) Representative: **Zacco Norway AS
P.O. Box 488 Skøyen
0213 Oslo (NO)**

(54) **DRYING OF FINE SILICON POWDER IN AMBIENT AIR BY EMPIRICALLY ESTABLISHING POWDER REACTIVITY**

(57)     The present invention relates to a method of drying powders which are reactive toward oxygen gas at industrial scale where oxygen gas is present in the atmosphere, while avoiding runaway heat development and excessive oxidation of the powder. This is achieved by empirically determining the reactivity of the powder by drying a smaller sample of powder at process parameters comparable to production scale and monitoring the degree of reaction. The invention is developed for silicon kerf-loss saw-dust powder from wafering of solar cells ("kerf"), but it is equally relevant for other fine silicon powders or reactive powders of other materials.

Figure 1: Illustration of powder arrangement in reactivity test container (c, top), larger production scale container (C, middle), heated area in a non-stationary continuous industrial scale process (bottom). "A/a" indicates the area of the heated volume base, while "H/h" indicates the height of the heated powder arrangement. The arrows indicate that the powder is transported through the stationary heated zone, indicated by the dashed lines.

EP 4 438 984 A1

**Description**

Field of the invention

The present invention relates to a method of drying powders which are reactive toward oxygen gas at industrial scale where oxygen gas is present in the atmosphere, while avoiding runaway heat development and excessive oxidation of the powder.

Background of the invention

In the solar and electronic industries, the demand for exceptional quality renders relatively high-quality high-value side streams unusable, and they are discarded. Ingots are often cropped into proper dimensions, giving saw dust. Furthermore, wafers are cut from the ingots by means of a diamond wire saw. Despite the very thin cutting wire of approximately 50 $\mu$m, the resulting kerf width of the cut is not much narrower than the wafers themselves. As such, the amount of kerf loss is approximately 40 % of the starting ingot volume. For both cropping and wafering, this kerf loss or "saw dust" (here labelled "kerf") is in the form of a very fine powder which is discarded to landfill or downcycled to low-value applications.
In 2019, the global annual production of solar cells was 129 GW (ref. https://www.statista.com/statistics/278917/global-solar-cell-production/). The generated amount of kerf from solar cell wafering can then be estimated using the following assumptions:

- Each cell is 150 $\mu$m thick.
- 40 % kerf is generated.
- All solar capacity comes from Si cells.
- The energy density used for sunlight when calculating annual capacity production is 1 kW/m$^2$.
- Each cell is 20 % efficient.

The resultant global amount of generated kerf from solar cell production was 160 000 tons in 2019, and the production capacity is increasing.
Use of kerf in high value applications has large commercial potential, but an important challenge is drying of the kerf. The watering process occurs in water, and the kerf is collected as a moist filter cake typically containing 20-60 % water. The first step toward utilization of the kerf as a raw material of higher value and quality, is its successful drying at competitive cost without introducing a high degree of oxidation in the process.
The kerf generated by diamond wire sawing is very fine. The mean particle diameter varies depending on the wafering process, from 700 nm to 2000 nm according to laser scattering particle size distribution measurements. The particles are highly anisotropic, however, and the BET specific surface area of 20-30 m$^2$/g corresponds to a diameter of 100 nm if the particles were spherical. The very small particle size makes the powder difficult to process in traditional industries. The powder is very reactive towards air and water upon heating according to the following reactions:

$$Si + O_2 \rightarrow SiO_2 \; \Delta H^{\ominus} = -911 \text{ kJ/mol}$$

$$Si + 2H_2O \rightarrow SiO_2 + 2H_2 \; \Delta H^{\ominus} = -339 \text{ kJ/mol}$$

These reactions occur spontaneously even at room temperature, but the generated $SiO_2$ forms a passivating layer on the surface, making the reaction rate temperature dependent. As the thickness of the oxide layer increases, the reaction rate at a constant temperature decreases. With increasing temperature, however, the reaction rate increases. Furthermore, either $O_2$ or $H_2O$ is required as a reactant for the oxidation. In the case of $H_2O$, its presence limits the maximum developed heat due to its evaporation. As long as $H_2O$ is present, evaporation will limit the temperature to roughly 100 °C. For runaway reaction and fire, $O_2$ is therefore the primary reactant of significance.
Finally, the specific surface area, *SSA,* (particle surface relative to particle mass) is important because the reaction occurs at the surface.

$$SSA = \frac{6}{\rho} \cdot \frac{1}{d} = \frac{6}{2.33 \text{ g/cm}^3} \cdot \frac{1}{d} = 2.58 \cdot 10^{-6} \text{ m}^3/\text{g} \cdot \frac{1}{d}$$

where $\rho$ is the density of silicon, and *d* is the diameter of a spherical particle.
*SSA* for spherical silicon particles of 10 $\mu$m, 1 $\mu$m, and 100 nm is 0.258 m$^2$/g, 2.58 m$^2$/g, and 25.8 m$^2$/g,

respectively. In other words, kerf with specific surface area corresponding to 100 nm spherical particles is 100 times more reactive than a powder with particle size 10 $\mu$m.

Kerf from wafering of solar cells and electronic wafers currently have *SSA* of more than 10 m$^2$/g, and experience shows that these powders easily catch fire during drying processes with industrially relevant conditions. Several trials made by the present inventors with heating the kerf under atmosphere containing ambient oxygen, even at moderate drying temperatures of around 100 °C, have resulted in self-ignition and subsequent uncontrollable thermal runaway oxidation of practically the entire mass of particulate silicon producing temperatures well above 1000 °C. The challenge is one of scale. The developed heat in the kerf is proportional to the amount of kerf, but the transport of that heat away from the kerf is much faster at smaller sample sizes. For this reason, the challenge of runaway thermal reactions may not be apparent before scale-up to industrial production.

The kerf loss saw dust silicon powder ("kerf") resulting from wafering of photovoltaic cells and electronic wafers represent a significant commercial potential for applications in high value markets, but drying of the moist powder is difficult or expensive at an industrial scale due to the reactivity. Various kerf materials show different levels of reactivity depending on the wafering process and further storage and treatment conditions. For these reason, optimal drying parameters vary depending on the material.

In the prior art (ref. CN 111 829 293 A, CN 111 302 345 A, CN 202 126 162 U, CN 110 745 828 A, and CN110081 880), expensive and cumbersome protective measures to suppress contact with ambient oxygen such as use of an inert atmosphere, vacuum drying, etc. have been suggested.

The present invention is based on the realization that some degree of oxidation of the silicon is usually unavoidable and can be accepted as long as the degree of exothermic heat development in the particle mass is prevented from heating up the bulk of the powder to such an extent that it sufficiently increases the reaction rate, causing an uncontrolled oxidation/thermal runaway. Thus, a safe (i.e. no thermal runaway) drying process relies on the rate of heat development and the rate of heat loss from the bulk phase of the particles to its surroundings. As long as the particles are cooled sufficiently to avoid a dangerous heating of the powder bulk, the drying process is safe.

However, as mentioned, the rate of oxidation is a function of the specific surface area, the powder temperature, and the degree of prior oxidation. A material with smaller particles and/or thinner surface oxide layer, will generate a larger amount of heat at a given process temperature and oxygen partial pressure. The specific surface area and oxidation level of kerf from different sources may vary significantly, making it necessary to adapt the drying process depending on the material.

The present invention addresses this issue in adjusting drying parameters to maximize production rate while ensuring a sufficient heat transport in relation to the reactivity of the material.

The present inventors have surprisingly been able to provide a method enabling cheap large scale safe drying of reactive powders (such as silicon kerf from wafering) by empirically deciding the reactivity of the powder in order to adjust the drying process parameters such that the heat developed by oxidation of silicon is less than the available heat transport of the system.

While the method was invented using kerf, it is equally relevant for other powders which are reactive upon heating developing a passivating surface layer. One example is coke.

Summary of the invention

It is a main object of the present invention to provide a new method of drying powders which are reactive towards oxygen gas at industrial scale where oxygen gas is present in the atmosphere, while avoiding runaway heat development and excessive oxidation of the powder.

Another object of the present invention is to provide a new method for safe drying of reactive silicon kerf and other powders becoming reactive upon heating under exposure to air, avoiding use of expensive and cumbersome protective measures to suppress contact with ambient oxygen.

These and other objects are obtained by subject-matter as defined in the accompanying claims.

Definitions

It is to be understood that the herein disclosed invention is not limited to the particular component parts of the material described or steps of the methods described since such material and method may vary. It is also to be understood that the terminology used herein is for purpose of describing particular embodiments only and is not intended to be limiting.

It should be noted that, as used in the specification and the appended claims, the articles "a", "an", "the", and "said" are intended to mean that there are one or more of the elements unless the context explicitly dictates otherwise. Thus, for example, reference to "a unit" or "the unit" may include several means, and the like. Furthermore, the words "comprising", "including", "containing" and similar wordings does not exclude other elements or steps.

The terms "powder", "kerf", or "material" as used herein consists of powder material wherein the particles can be free flowing or bonded into agglomerates, pellets, briquettes, lumps, filter cake, green bodies etc.

The terms "kerf-loss saw-dust", "silicon kerf", "kerf", and "kerf particles" are used interchangeably in the present disclosure for the small silicon particles formed during silicon cutting or abrasive processing such as for example diamond wire cutting of blocks into wafers.

The term "d50" or "$d_{50}$" as used herein means the particle mass-median-diameter.

The terms "container(s)" and "pan(s)" are used interchangeably in the present disclosure and include containers/pans of any geometrical form such as rectangular, prismatic, cylindrical, spherical etc. in which the material is placed for purpose of drying.

The terms "pathway of efficient heat conduction" and "feature of heat removal" are used interchangeably in the present disclosure and means e.g. the surface of the powder exposed to air maintaining the process temperature, or an internal heat conductive material such as a container wall maintaining the process temperature.

The terms "characteristic distance" and "heat removal distance" ("d") are used interchangeably in the present disclosure and means the heat transfer distance from a volume of powder, through the adjacent powder, to the closest feature of heat removal.

The term "over-temperature" is used in the present disclosure to describe the difference in temperature between a volume of powder and the surroundings due to powder self-heating.

"Comparable" is used in the present disclosure when describing parameters in test and production scales. Comparable is then understood to mean conditions that are in the same order of magnitude so that relevant mechanisms such as over-temperature are present in both cases. Furthermore, these mechanisms must respond relatively similar to process changes such as process temperature and characteristic distance in both cases. In particular, the reactivity test must be on a large enough sample that the thermal insulation of the powder is sufficient to enable and quantify the exothermic reaction due to self-heating.

"Process temperature" is used in the present disclosure to mean nominal setpoint drying temperature. Furthermore, the actual process temperature is maintained at nominal value unless otherwise stated. During drying without significant powder reaction, the powder temperature is equal to, or lower than the process temperature so that heat is transported into the powder. During significant powder reaction and heat production, the powder temperature may be higher than the process temperature so that heat is transported out of the powder. The process temperature therefore represents the potential for both heating and drying of the moist powder, and the cooling during powder heat development.

The term "temperature profile", abbreviated "tp" and "TP", is used in the present disclosure to indicate that the process temperature is, or may be, variable as a function of time.

The terms "reactivity test", "small scale", "sample scale", "test scale", "test", and "sample" are used interchangeably in the present disclosure meaning the drying of a sample of powder in order to determine its reactivity and relative probability of runaway reaction. The reactivity test is strictly defined in the description of the invention.

The terms "industrial scale", "production scale", "production" and "commercial" are used in interchangeably the present disclosure meaning drying of a large enough volume of powder for it to be commercially viable.

The terms "parameter(s)", "process parameters", and "conditions" are used in the present disclosure to collectively refer to process conditions such as atmosphere content, air temperature, air flow, pressure, process temperature, power of heat source, powder arrangement, container dimensions etc.

Brief description of the figures

Figure 1: Illustration of physical powder arrangements.

Figure 2: Illustration of heat removal distance for a selection of geometries.

Figure 3: Reactivity test on coarse silicon powder.

Figure 4: Process monitoring during drying of kerf at too high temperature relative to kerf reactivity.

Figure 5: Image of powder producing significant heat during runaway reaction.

Figure 6: Reactivity test on kerf from same source and production period as the runaway reaction in industrial scale in Figures 4 and 5.

Figure 7: Reactivity test of one of many sources successfully dried without incidents.

Figure 8: Reactivity test of kerf.

Figure 9: Reactivity test of the material in Figure 8 after modification.

Figure 10: Reactivity test of a source dried without incidents.

Figure 11: Reactivity test of the same source as in Figure 10 at higher process temperature.

Figure 12: Measured temperatures in centre of samples of different physical layouts with and without internal heat-conductive features.

Detailed description of the invention

According to the invention, there is provided a method of drying powder at industrial scale, comprising the following steps:

1) Reactivity test: determining reactivity of a sample of a powder by

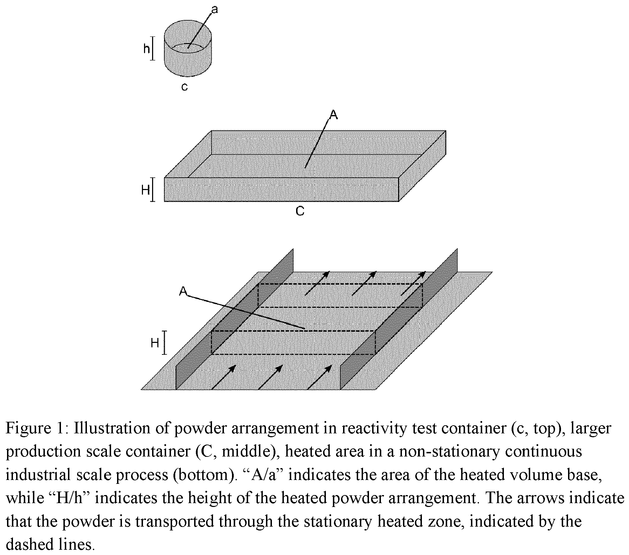

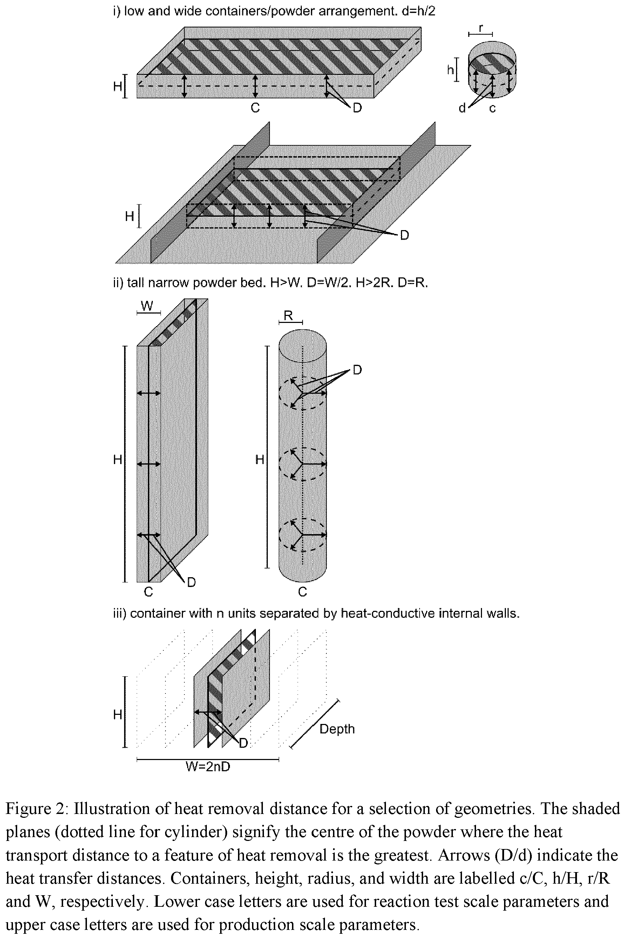

    a. providing a sample of a moist powder,
    b. placing the sample in a container (c in Figure 1),
    c. defining a characteristic distance (d in Figure 2) which is the shortest heat transfer distance from the centre of the sample to a feature of heat removal,
    d. heating the sample under atmosphere containing oxygen at a controlled temperature profile (tp), while measuring the reactivity of the powder sample, and

2) Industrial scale: drying by

    a. arranging an industrial scale charge of the moist powder with a characteristic distance (D in Figure 2) which is the shortest heat transfer distance from the centre of the powder to a feature of heat removal comparable to the characteristic distance (d) in step 1c,
    b. heating the powder under atmosphere containing oxygen following a controlled temperature profile (TP) comparable to tp in step 1d,
    c. wherein D and TP are decided based on the reactivity of the powder sample determined in steps 1a-d.

These aspects of the invention are described in more detail in the following:

- General process design considerations applicable to both reactivity test and production scales.
- The design and process parameters of the reactivity test.
- The design and process parameters of the industrial scale drying.
- The relationship between reactivity test parameters and industrial scale parameters.

**General process design considerations applicable to both reactivity test and production scales**

- Powder material for which the method is applied:
  The powder to be subjected to the method of the invention is reactive towards oxygen upon heating and forms a passivating layer upon oxidation. Any heat sensitive particles prone to uncontrolled oxidation when exposed to ambient air at temperatures higher than the ambient temperature is applicable for drying in the present method.

In a preferred embodiment of the invention, the powder to be dried is of silicon kerf.
In an embodiment of the invention, the powder to be dried is of silicon with dry basis purity above 80 %, more preferably above 90 %, and more preferably above 95 %.
In an embodiment of the invention, the specific surface area of the powder (*SSA*) is more than 1 $m^2$/g, and preferably more than 10 $m^2$/g.
In an embodiment of the invention, the specific surface area of the powder (*SSA*) is less than 100 $m^2$/g, and preferably less than 50 $m^2$/g.
In an embodiment of the invention, the mass-mean-diameter (d50) of the particles according to laser scattering is between 10 nm and 10 $\mu$m, preferably between 50 nm and 5 $\mu$m, and more preferably between 100 nm and 2 $\mu$m.

In another embodiment, the powder to be dried is of a material different than silicon, preferably a material reactive toward oxygen exhibiting a time-limited exothermic reaction when the powder temperature increase is not too great during self-heating.

-Heating method:

The heating is carried out by means of one or more of circulating heated gas, resistive heating of the container, infrared irradiation, microwave irradiation, visible light irradiation, or other methods.

In a preferred embodiment, the powder is heated by circulating heated air.

In an embodiment, the powder is heated by irradiation of infrared/ultraviolet/visible light or microwaves.

In an embodiment, the powder support (i.e. the material on which the powder is arranged such as the bottom of a container or a non-stationary device) is heated directly by resistive heating.

-Atmosphere content:

In a preferred embodiment, ambient air is used as process atmosphere.

The content of the process atmosphere is influenced by the process.

In an embodiment, oxidation of the powder reduces the actual oxygen partial pressure of the process atmosphere to 0.02 atm or more, preferably to 0.05 atm or more, more preferably to 0.1 atm or more, and even more preferably to 0.15 atm or more.

In an embodiment, the ambient air is diluted by evaporated water by up to 100 % water, preferably by up to 80 % water, more preferably by up to 50 % water.

-Process temperature:

The process temperature should be adapted to the powder reactivity and the characteristic distance of the powder arrangement. In the reactivity test, the temperature must be high enough that the powder heat production is detected unless the powder is relatively inert. In general, the temperature should be high enough to allow drying at a profitable production rate.

In one embodiment, suitable for a large number of kerf qualities, the process temperature is in the range from 50 °C to 300 °C, preferably in the range 110 °C to 200 °C, more preferably in the range 120 °C to 180 °C.

In one embodiment, the process temperature of the reactivity test is higher than or equal to the process temperature of production scale.

-Drying duration:

The drying duration is dependent on process temperature, characteristic distance, initial moisture, particle size, surface properties, target moisture etc. The reactivity test duration should be until the powder reactivity has been quantified (depending on method of reactivity determination, as further described in this section). Production drying duration should be until the product moisture is within specification.

In a preferred embodiment for batch processing, the heating lasts from 1 day to 7 days, or preferably from 1.5 days to 4 days.

In an embodiment for continuous drying, the duration within the heated zone is 10 min to 10 hours, preferably from 30 min to 2 hours.

-Physical design of powder arrangement:

To avoid a high level of over-temperature and a corresponding high probability of runaway reaction, the main feature of significance in the physical arrangement of the powder is the characteristic distance from the centre of the powder to a pathway of efficient heat conduction (d/D). To avoid an increasing powder temperature, the transferred heat must be equal to the produced heat. Heat conduction through a homogeneous material is given by the simple formula

$$q = -k \frac{\Delta \mathrm{T}}{d}$$

, where $q$ is heat flux (W/m$^2$), $k$ is the thermal conductivity, $\Delta$T is the temperature difference, and $d$ is the distance related to the temperature difference (the characteristic distance). In other words, for a given material, the over-temperature is inversely proportional to the characteristic distance.

For this reason, this characteristic distance (d, D) is the key feature that must be comparable between production setup and the reactivity test design.

Figure 1 shows illustrations of some example powder arrangements for drying at industrial and sample scales, while Figure 2 shows illustrations of the characteristic distance (d/D) in various physical layouts. Said distance may also be named "heat removal distance" as it indicates the characteristic length for which heat transport must occur for a given geometry of powder arrangement. The feature of heat removal, which is the endpoint of the distance (d, D), may be the closest surface exposed to air flow, an external heat conductive material such as the container bottom or walls, or an internal heat conductive material such as a container divider wall. In Figure 2, arrows indicate characteristic distances D and d. The shaded areas and the dotted line indicate the centre of the powder (i.e. the starting point of the heat conduction).

In one embodiment, the feature of heat removal is the top surface of the powder exposed directly to air and/or the side and bottom surface of the powder exposed to the air temperature through the walls and bottom of the container.

In a second embodiment, the feature of heat removal is an internal heat conductive material such as a metal wall, plate, rod, spike, pipe etc.

In a third embodiment, the feature of heat removal is partly or completely comprised of a belt, slide, or other support for the powder being transported through a heated zone.

-The obtained product (i.e. dried silicon powder):

This product is useful as a general source of silicon in traditional or novel applications.

In one embodiment, the material is sold on the open market as an alternative to traditional metallurgical grade silicon or ferrosilicon.

In another embodiment, the material is sold to applications where the small particle size and/or high purity are features of added value.

In another embodiment, the drying process described here is employed as a sub-part of a production process of several steps.

In another embodiment, applying the method to a material different to silicon, the product is used for traditional or novel applications.

**Design and process parameters of the reactivity test**

The reactivity of the powder material is measured during drying of a sample of the material in a much smaller setup than industrial scale. The process parameters such as temperature, atmosphere, and the shortest length of heat transport from the centre of the powder are comparable for the sample and industrial setups.

-Method of reactivity quantification:

The reactivity of the powder sample can be measured by one or more of the following means:

    i. Measuring temperature (T) in bulk of the sample at one or more locations, and relating reactivity to the observed over-temperature.

    ii. Measuring the power of the temperature-controlled heat source and assigning observed relative drop in power to heat produced by the powder.

    iii. Measuring the process temperature in a power-controlled heat source and assigning relative increase in temperature to powder reactivity.

    iv. Measuring oxygen consumption in process gas, and relating drop in $pO_2$ to oxidation of silicon/other material (i.e. the powder).

    v. Measuring developed amount of hydrogen, and relating measured $H_2$ to water oxidation of silicon/other material (i.e. the powder).

    vi. Measuring mass change of the sample, and comparing mass loss to a known reference moisture number so that a positive deviation can be related to oxygen bonded to silicon/other material (i.e. the powder).

    vii. Measuring mass loss during drying and comparing rate of heat loss (water evaporation) to applied power, and relating a relative higher rate of evaporation to heat generated by powder.

In a preferred embodiment, the reactivity is determined based on the magnitude of the over-temperature measured in the centre of the powder.

In another embodiment, the powder reactivity is measured by monitoring the partial pressure of oxygen while introducing a controlled amount of oxygen to the process.

In another embodiment, the powder reactivity is measured by monitoring the amount of generated hydrogen.

In another embodiment, the powder reactivity is measured by monitoring the weight of the powder. If the drying rate is higher than applied energy would suggest, one can calculate the heat generated in the powder. If the final mass is higher than the expected final mass, the reactivity can be calculated from the mass increase of oxygen absorption according to $Si + O_2 \rightarrow SiO_2$.

In another embodiment, the powder reactivity is measured by monitoring the power consumed by the process heating relative to a known base consumption. When consumed energy is lower than expected, the difference corresponds to the heat produced by the powder.

-The shape of the reactivity test container:

The container (c, Figure 1) used for the reactivity test has unlimited possible physical shapes and dimensions. The only required feature is that the characteristic distance from the centre of the powder to a pathway of efficient heat transfer (d) must be comparable to the characteristic distance in the industrial scale (D). The heat conductivity through the powder itself is an important parameter influencing the probability of runaway reaction.

In a preferred embodiment, the container (c) is cylindrical with height smaller than the width/diameter so that

$$d = \frac{h}{2}$$.

In another embodiment, the container (c) is cylindrical with height larger than the diameter so that d = r (radius).

In another embodiment, the container (c) is a spherical container of perforated material such that air and steam can pass, while the powder is kept inside.

-The size of the reactivity test:

The reactivity test must be on a large enough sample that the thermal insulation of the powder is sufficient to enable and quantify the powder exothermic reaction due to self-heating.

In one embodiment, the volume of the reactivity test sample is between 100 ml and 10 l, preferably between 0.5 l and 5 l, more preferably between 1 l and 5 l.

-The characteristic distance, d:

The characteristic distance, d, must be comparable between the reactivity test and production scale drying.

In one embodiment, 0.25D < d < 5D, preferably 0.25D < d < 2D, more preferably D < d, where D is the characteristic distance of production scale, and d is the characteristic distance of test scale.

In one embodiment, d is in the range 1-50 cm, preferably in the range 2.5-20 cm, and more preferably in the range 5-10 cm.

**The design and process parameters of the industrial scale drying**

-The physical powder arrangement:

In a preferred embodiment, the powder is arranged in one or more containers (C) such as pans of solid bottom and walls having height (H) and characteristic distance ( $D = \frac{H}{2}$ ).

In another embodiment, the moist powder is arranged in a non-stationary fashion and transported through a heated zone, for example on a belt that may be moving (i.e. continuous drying).

-The feature of heat removal:

In a preferred embodiment, the powder is arranged with a height (H) which is smaller than the width/depth of the container or heated zone, so that the feature of heat removal is the top surface exposed to air, and indirectly through the bottom support exposed to air.

In another embodiment, the powder is arranged in container(s) (C) of solid bottom and walls and internal vertical walls. The distance between the vertical walls is less than the container height, so that D is half the distance between the walls as illustrated in Figure 2iii.

In another embodiment, the container(s) (C) is equipped with other internal temperature equalising features such as plates, walls, fins, rods, or tubes. The cooling features may be of e.g. aluminium or steel, preferably placed equally spaced vertically in the container(s).

-The size and capacity of the industrial scale drying:

In a preferred embodiment, the batch size of the industrial set-up has a volume (V) that is much larger than the volume (v) of sample scale container (c), i.e. V»v.
In a preferred embodiment, the batch size of the production scale drying is more than 50 times larger than the reactivity test size, more preferably more than 100 times larger.
In a preferred embodiment, the production scale capacity of a single container (of which several may be heated in parallel) has a capacity of at least 5 times the container of the reactivity test, more preferably more than 10 times the container of the reactivity test.
In a continuous drying process, a preferred embodiment has a dry product capacity of greater than 10 kg/hour, preferably greater than 20 kg/hour, more preferably greater than 50 kg/hour.

-The characteristic distance, D:

In a preferred embodiment, the characteristic distance is as short as possible, preferably less than 10 cm, more preferably less than 5 cm.
For a continuous process, the characteristic distance, D, is preferably shorter than for a batch process, preferably shorter than 5 cm, more preferably shorter than 2 cm, and even more preferably shorter than 1 cm.

**The relationship between reactivity test parameters and industrial scale parameters**

In a first embodiment, production parameters known to be safe from trial and error on material A is used for material B when reactivity test results are similar for materials A and B, or if B is found to be less reactive than A.
In a second embodiment, production parameters known to be safe from trial and error on material A are changed by a factor x for material B, when the same factor x change in reactivity test parameters provided similar results for the materials in small scale.
In a third embodiment, reactivity tests are conducted on a material at different process parameters yielding various reactivity results. Production parameters are set equal to the test parameters where the reactivity was less than a pre-defined value.
A fourth embodiment is equal to the third embodiment, with an added safety margin by a given reduction of process temperature or characteristic distance in production scale.
The invention is explained in more detail in the examples below. The examples are only meant to be illustrative and shall not be considered as limiting.

Examples

A number of experimental results are presented in the following, demonstrating a clear relationship between controlled drying (i.e. no runaway reaction) and the measured reactivity of the same powder at small scale.

**Explanation of reactivity tests**

All reactivity tests presented here were conducted by placing a sample of powder in an open container constructed such that the shortest distance from the centre of the container to any outside surface (air on top, steel container at bottom) was straight up or down. That is, the height of the container was less than its width and/or depth. Four temperature sensors were fixed permanently at the centre column of the container, at different distances from the top and bottom. These locations were the same for all reactivity tests. Unless specified otherwise, the container in the reactivity tests was cylindrical with height 10 cm and radius 10 cm. The process temperature of each experiment is noted in the figure texts. The sample container was placed in a heating cabinet providing controlled temperature and airflow.
Any over-temperature relative to process temperature measured in the bulk of the sample was due to the powder itself producing heat. However, because the sample was relatively small compared to the ability of the heating cabinet to absorb and dissipate this heat, the surrounding temperature was not affected much, and the powder generally cooled down after a period of reaction.
A similar over-temperature in the industrial scale does not have the same result because the industrial scale drying chamber has a relatively much larger charge of powder relative to the heat dissipation ability. For example, one may calculate the heat production resulting from the oxidation of 1 % of the silicon mass. This is reasonable, as the surface oxide layer on the particles is already somewhere between 1 and 5 % of the powder mass. In a sample of 2 kg this gives 0.158 kWh which, if spread over 30 minutes, means 0.316 kW of produced heat. This is easily dissipated in the equipment. In production however, the mass of powder may be 2 tons. This means a heat production of 316 kW which is not easily dissipated. Granted, the entire mass does not react simultaneously, but the number is still many times more than what the drying process requires. For this reason, the process air temperature will increase, causing an even higher temperature for the silicon oxidation, causing an even higher rate of reaction, eventually ensuring that most material is reacting and producing heat.

In short: the probability of runaway reaction in industrial scale is directly related to the over-temperature observed in reactivity tests, even when runaway reaction does not occur in the reactivity test. While the actual produced heat in kWh/kg could be estimated from the reactivity tests, this was not the employed method. Rather, the reactivity was considered relatively to other samples.

Example 1 - Coarse powder

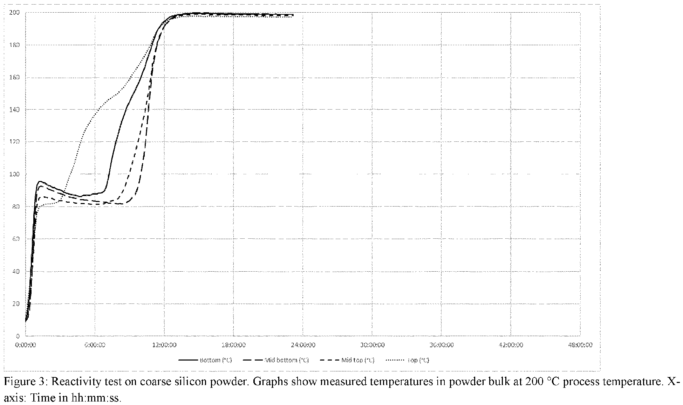

The result of the reactivity test of a coarse Si powder from another type of process than wafering is shown in Figure 3. For this material, d50 was more than 5 $\mu$m, and the heat developed due to reaction of the powder was none or negligible, as can be seen from the absence of any over-temperature in the reactivity test. That is: the powder was never measured to be at higher temperature than the surrounding process temperature. This quality of powder was safely dried at industrial scale where D was greater than d, but smaller than 2d. The process temperature in industrial scale was comparable to the process temperature used in the test (0.75tp < TP < 1.25tp).

Example 2 (Reference Example) - Runaway reaction at industrial scale

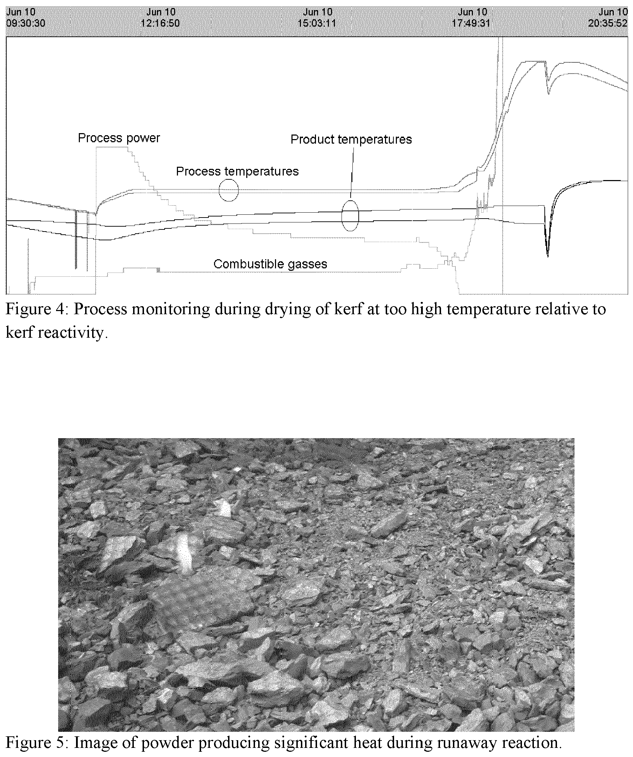

In this production, the raw material had not been investigated by the reactivity test, and the process parameters were too aggressive. Figure 4 shows the process monitoring graphs during an incident of runaway reaction during drying at industrial scale. The power was automatically adjusted to maintain the correct process temperature, and prior to 16:45 the power was relatively constant, corresponding to evaporation energy and process heat losses. After 16:45, however, the measured power started decreasing due to the emergence of another significant heat source (kerf oxidation). At approximately 17:30, this heat source was more powerful than the required process power, causing the air temperature to increase above the process set temperature despite the process heat source no longer producing power. Simultaneously, some combustible gas was produced, indicating incomplete combustion of organic impurities or development of $H_2$ gas. Both combustible gas alternatives suggest some area of high temperature and low atmosphere concentration of Oz. At this point, the powder containers were removed from the drying chamber, and embers and flames were observed in a small number of the containers. One of these are shown in Figure 5.

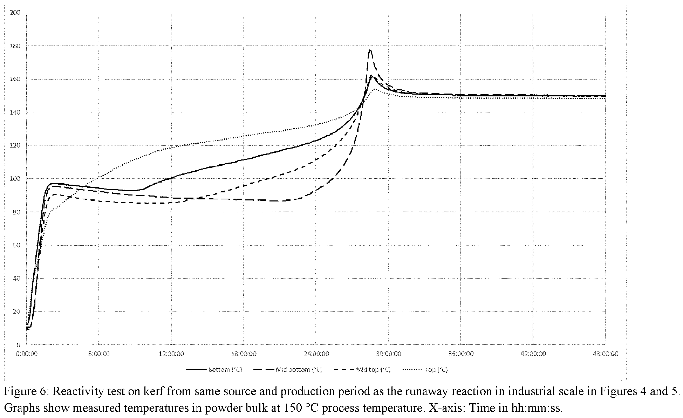

A reactivity test was performed on the material after the fact, as shown in Figure 6. It can be seen that during drying at constant process air temperature of 150 °C, the powder reached temperatures of up to 180 °C, an over-temperature of 30 °C. The industrial scale process parameters were comparable to the process parameters of the reactivity test. The characteristic distance and process temperature of the industrial scale process was greater than 75 % and below 150 % of the corresponding values of the reactivity test. The production scale batch size was roughly 1000 times greater than the reactivity test.

Example 3 - Source dried at industrial scale with no incidents of runaway reaction

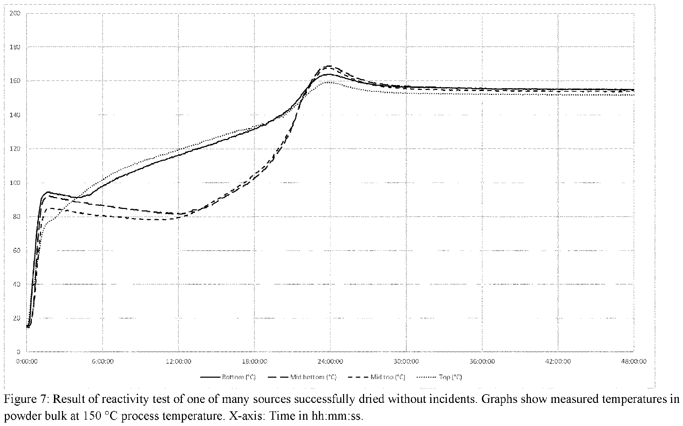

Figure 7 shows the result of a reactivity test of another kerf powder that was successfully dried without incidents at industrial scale. The parameters of both reactivity tests and industrial scale drying were equal to that of Reference Example 2, but the measured over-temperature in Figure 7 was 20 °C, demonstrating that the difference between 20 °C and 30 °C over-temperature in equal reactivity tests signified if the material was safe or unsafe at the given production process parameters.

Example 4 - Kerf material modified to reduce reactivity

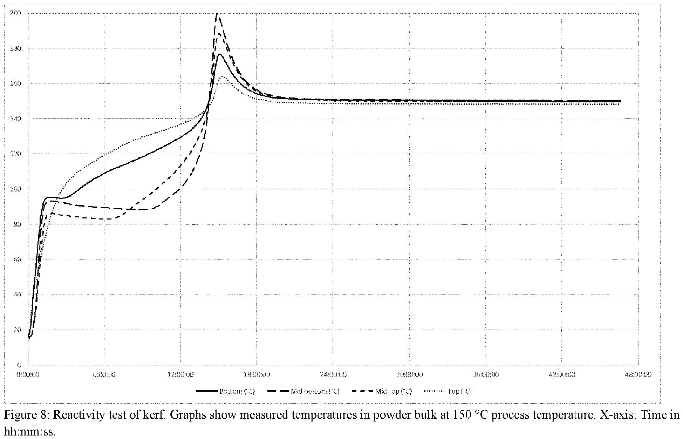

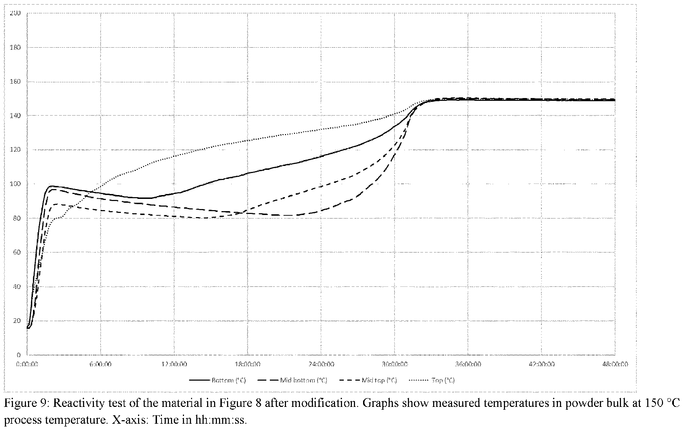

A shipment of another kerf quality was analysed to be relatively reactive, as shown in Figure 8. For this material, a treatment was made in order to reduce the reactivity to the level as shown in Figure 9. This resultant quality was then successfully dried at a scale of 10 tons. The process parameters of the industrial scale drying of this material was comparable to that of the reactivity test.

Example 5 - Source where production temperature was increased

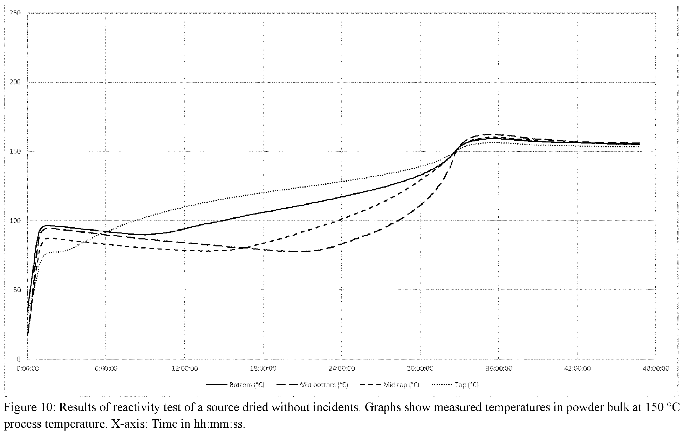

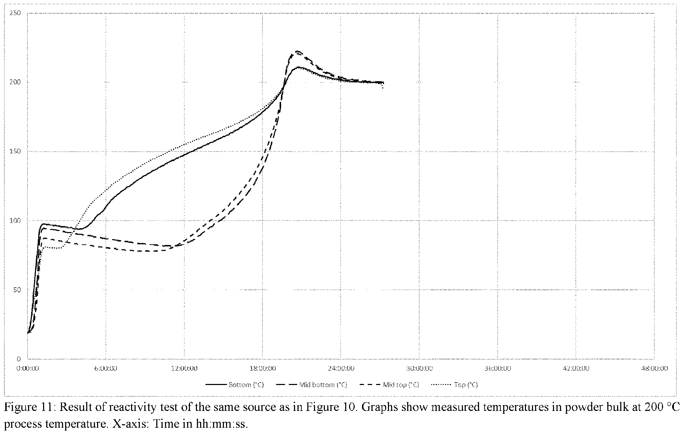

Figures 10 and 11 show the reactivity tests of one sample at 150 °C and 200 °C. At 150 °C the over-temperature was minor, while at 200 °C it was less than 30 °C. Due to the relatively low measured reactivity, this material was dried at 40 °C higher temperature than what was used in Examples 2-3. No incidents occurred.

Example 6 - Internal heat-conducting pathways

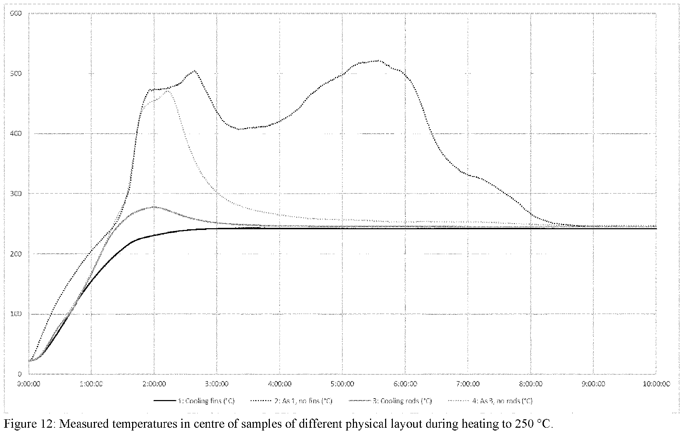

As explained above, the characteristic distance (d, D) is a key feature that must be comparable between production setup and the reactivity test design. In the following, an experiment proving the importance of this is presented.
Four samples were arranged in different physical setups. Sample 1 was placed in a container containing vertical aluminium cooling fins. Sample 2 had the same outer dimensions as Sample 1, but did not contain any internal features. The amount of powder was equal (520 g) in order to keep the comparison relevant with respect to production capacity. For this reason, the sample height of sample 2 was lower than that of sample 1. The distance between the cooling fins in sample 1 was significantly less than the height of sample 2.
Samples 3 and 4 had equal outer dimensions and sample mass (475 g), but the values were different to that of samples 1 and 2. In sample 3, steel tubes of 10 mm outer diameter were placed equally spaced vertically in the container such that the distance between the tubes were significantly less than the height of the container. Temperature measurement probes were placed in the centre of all samples, and the temperature was logged as heated air of 250 °C was applied, as shown in Figure 12. 250 °C is much higher than what samples of this reactivity and sample sizes normally can withstand without runaway reaction, and the cooling fins and steel tubes were clearly efficient at dissipating the heat, while samples 2 and 4 showed significant oxidation. Thus, the internal heat conductive features of samples 1 and 3 were clearly effective in reducing the oxidation and temperature increase due to the shorter characteristic distances.
As demonstrated by the examples, the method according to the present invention enables applying as high drying temperatures as possible while maintaining only the required safety margin toward runaway reaction. That is, to maximize production rate while maintaining a material specific tailored safety margin.
The person skilled in the art realizes that the present invention is not limited to the preferred embodiments described above. The person skilled in the art further realizes that modifications and variations are possible within the scope of the appended claims. Additionally, variations to the disclosed embodiments can be understood and effected by the skilled person in practicing the claimed invention, from a study of the disclosure, and the appended claims.
The projects leading to this patent application have received funding, from the European Union's Horizon 2020 research and innovation programme under grant agreements No 958365, No 641972, No 875568, from the Research Council of Norway (SkatteFUNN) and from EIT Raw Materials GmbH (Agreement no 17120).

**Claims**

1. A method of drying powder at industrial scale, comprising the following steps:

   1) Reactivity test: determining reactivity of a sample of a powder by

      a. providing a sample of a moist powder,
      b. placing the sample in a container (c),
      c. defining a characteristic distance (d) which is the shortest heat transfer distance from the centre of the sample to a feature of heat removal,
      d. heating the sample under atmosphere containing oxygen at a controlled temperature profile (tp), while measuring the reactivity of the powder sample,

   and
   2) Industrial scale: drying by

      a. arranging an industrial scale charge of the moist powder with a characteristic distance (D) which is the shortest heat transfer distance from the centre of the powder to a feature of heat removal comparable to the distance (d) in step 1c,
      b. heating the powder under atmosphere containing oxygen following a controlled temperature profile (TP) comparable to the tp in step 1d, and
      c. wherein D and TP are decided based on the reactivity of the powder sample determined in steps 1a-d.

2. The method according to claim 1, wherein the characteristic distance (D) is comparable to the characteristic distance (d) in that 0.25D < d < SD.

3. The method according to claim 1 or 2, wherein the feature of heat removal is a surface of the powder exposed to air, an external heat conductive material such as bottom or wall(s) of the container (c) or in/on which the industrial scale charge of the powder is arranged, or an internal heat conductive material of the container (c) or in/on which industrial scale charge of the powder is arranged such as one or more divider walls, plates, rods, spikes or pipes.

4. The method according to claim 3, wherein the industrial scale charge of the powder is arranged in one or more containers (C), or in a non-stationary manner such as on a moving belt.

5. The method according to any preceding claims, wherein the heating in the reactivity test and the industrial scale is carried out by means of circulating heated gas; resistive heating of the external and/or internal heat conductive material(s), infrared irradiation; microwave irradiation; visible light irradiation; or mixtures thereof.

6. The method according to claim 5, wherein the heating is performed at a process temperature in the range from 50 °C to 300 °C.

7. The method according to any preceding claims, wherein the atmosphere containing oxygen in the reactivity test and the industrial scale is ambient air, or ambient air diluted by evaporated water.

8. The method according to any preceding claims, wherein the reactivity of the powder sample is measured by one or more of the following means:

   i. measuring temperature (T) in bulk of the sample at one or more locations, and relating reactivity to observed over-temperature,
   ii. measuring power of a temperature-controlled heat source and assigning observed relative drop in power to heat produced by the powder,
   iii. measuring process temperature in a power-controlled heat source and assigning relative increase in temperature to powder reactivity,
   iv. measuring oxygen consumption in process gas, and relating drop in $pO_2$ to oxidation of the powder,
   v. measuring developed amount of hydrogen, and relating measured $H_2$ to water oxidation of the powder,
   vi. measuring mass change of the sample, and comparing mass loss to a known reference moisture number so that a positive deviation can be related to oxygen bonded to the powder,
   vii. measuring mass loss during drying and comparing rate of heat loss (water evaporation) to applied power, and relating a relative higher rate of evaporation to heat generated by powder.

9. The method according to any preceding claims, wherein the powder is reactive towards oxygen upon heating.

10. The method according to claim 9, wherein the powder is of silicon kerf.

Figure 1: Illustration of powder arrangement in reactivity test container (c, top), larger production scale container (C, middle), heated area in a non-stationary continuous industrial scale process (bottom). "A/a" indicates the area of the heated volume base, while "H/h" indicates the height of the heated powder arrangement. The arrows indicate that the powder is transported through the stationary heated zone, indicated by the dashed lines.

i) low and wide containers/powder arrangement. d=h/2

ii) tall narrow powder bed. H>W. D=W/2. H>2R. D=R.

iii) container with n units separated by heat-conductive internal walls.

Figure 2: Illustration of heat removal distance for a selection of geometries. The shaded planes (dotted line for cylinder) signify the centre of the powder where the heat transport distance to a feature of heat removal is the greatest. Arrows (D/d) indicate the heat transfer distances. Containers, height, radius, and width are labelled c/C, h/H, r/R and W, respectively. Lower case letters are used for reaction test scale parameters and upper case letters are used for production scale parameters.

Figure 3: Reactivity test on coarse silicon powder. Graphs show measured temperatures in powder bulk at 200 °C process temperature. X-axis: Time in hh:mm:ss.

Figure 4: Process monitoring during drying of kerf at too high temperature relative to kerf reactivity.

Figure 5: Image of powder producing significant heat during runaway reaction.

Figure 6: Reactivity test on kerf from same source and production period as the runaway reaction in industrial scale in Figures 4 and 5. Graphs show measured temperatures in powder bulk at 150 °C process temperature. X-axis: Time in hh:mm:ss.

Figure 7: Result of reactivity test of one of many sources successfully dried without incidents. Graphs show measured temperatures in powder bulk at 150 °C process temperature. X-axis: Time in hh:mm:ss.

Figure 8: Reactivity test of kerf. Graphs show measured temperatures in powder bulk at 150 °C process temperature. X-axis: Time in hh:mm:ss.

Figure 9: Reactivity test of the material in Figure 8 after modification. Graphs show measured temperatures in powder bulk at 150 °C process temperature. X-axis: Time in hh:mm:ss.

Figure 10: Results of reactivity test of a source dried without incidents. Graphs show measured temperatures in powder bulk at 150 °C process temperature. X-axis: Time in hh:mm:ss.

Figure 11: Result of reactivity test of the same source as in Figure 10. Graphs show measured temperatures in powder bulk at 200 °C process temperature. X-axis: Time in hh:mm:ss.

Figure 12: Measured temperatures in centre of samples of different physical layout during heating to 250 °C.

Europäisches
Patentamt

European
Patent Office

Office européen
des brevets

# EUROPEAN SEARCH REPORT

Application Number

EP 23 19 8644

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 114 543 489 A (SHENZHEN JINHAOMIAO INDUSTRY DEVELOPMENT LTD COMPANY) 27 May 2022 (2022-05-27) * abstract; figures * | 1 | INV. F26B25/00 |
| A | CN 112 880 369 A (XIAN ESWIN SILICON WAFER TECH CO LTD; XIAN ESWIN MATERIAL TECH CO LTD) 1 June 2021 (2021-06-01) * abstract; figure * | 1 | |
| A | CN 110 686 466 A (WANG FENGYUN) 14 January 2020 (2020-01-14) * abstract; figures * | 1 | |
| A | CN 103 466 625 A (CHINA ENFI ENG CORP) 25 December 2013 (2013-12-25) * abstract; figures * | 1 | |
| A | JP 2017 062061 A (HITACHI CHEMICAL CO LTD) 30 March 2017 (2017-03-30) * abstract; figures * | 1 | |
| | | | TECHNICAL FIELDS SEARCHED (IPC) |
| A | CN 203 024 547 U (SINOPEC NANJING ENGINEERING & CONSTRUCTION INC ET AL.) 26 June 2013 (2013-06-26) * abstract; figure * | 1 | F26B G01N |
| A | CN 203 440 091 U (CHINA ENFI ENG CORP) 19 February 2014 (2014-02-19) * abstract; figures * | 1 | |
| A | CN 202 126 162 U (GUODIAN NINGXIA SOLAR CO LTD) 25 January 2012 (2012-01-25) * abstract; figures * | 1 | |
| A | NO 20 210 855 A1 (VIANODE AS [NO]) 3 January 2023 (2023-01-03) * the whole document * | 1 | |

The present search report has been drawn up for all claims

| Place of search | Date of completion of the search | Examiner |
|---|---|---|
| The Hague | 26 February 2024 | Van Dooren, Marc |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 23 19 8644

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2024

| Patent document cited in search report | | Publication date | Patent family member(s) | Publication date |
|---|---|---|---|---|
| CN 114543489 | A | 27-05-2022 | NONE | |
| CN 112880369 | A | 01-06-2021 | NONE | |
| CN 110686466 | A | 14-01-2020 | NONE | |
| CN 103466625 | A | 25-12-2013 | NONE | |
| JP 2017062061 | A | 30-03-2017 | NONE | |
| CN 203024547 | U | 26-06-2013 | NONE | |
| CN 203440091 | U | 19-02-2014 | NONE | |
| CN 202126162 | U | 25-01-2012 | NONE | |
| NO 20210855 | A1 | 03-01-2023 | NONE | |

EPO FORM P0459

For more details about this annex : see Official Journal of the European Patent Office, No. 12/82

**REFERENCES CITED IN THE DESCRIPTION**

**Patent documents cited in the description**

- CN 111829293 A
- CN 111302345 A
- CN 202126162 U
- CN 110745828 A
- CN 110081880